# EUROPEAN PATENT APPLICATION

(11) **EP 4 393 472 A1**
(43) Date of publication of application: **03.07.2024**
(21) Application number: 22216779.3
(22) Date of filing: 27.12.2022
(51) Int. Cl.: A61K 9/00, A61K 9/20, A61K 9/48, A61K 47/12, A61P 29/00, A61K 31/702

(54) **MANNURONATE, GULURONATE AND GULUMANNURONATE TABLET, CAPSULE OR AMPOULE FORMULATION AND METHOD FOR ITS PREPARATION**

(71) Applicant: Mirshafiey, Abbas, 14155 Teheran (IR); Lalander, Jacob, 1001 Riga (LV)
(72) Inventor: MIRSHAFIEY, Abbas, 14155 Teheran (IR); LALANDER, Jacob, LV-1001 Riga (LV)
(74) Representative: Maiwald GmbH

(57) **Abstract**

The present invention relates to tablet, capsule and ampoule formulations for oral and/or injectable use, the formulation comprising β-D-mannuronic acid or α-L-guluronic acid or a mixture of β-D-mannuronic acid and α-L-guluronic acid, oligomers thereof, or pharmaceutically acceptable salts thereof, wherein the total amount of β-D-mannuronic acid or α-L-guluronic acid, or their mixture, oligomers thereof, or pharmaceutically acceptable salts thereof is 10 wt.-% to 100 wt.-% based on the total weight of the tablet or capsule, or 20 wt.-% to 60 wt.-% based on the total weight of the ampoule formulation. Additionally, the invention relates to tablet, capsule and ampoule formulations for oral and/or injectable use, preferably as a supplement or medication, in a method for treating neurodegeneration, inflammatory reactions, pain, cancer, aging, diabetes, and/or cadiopathy, and methods for preparing such formulations.

## Description

### FIELD OF THE INVENTION

The present invention relates to tablet, capsule and ampoule formulations for oral and/or injectable use, the formulation comprising β-D-mannuronic acid or α-L-guluronic acid or a mixture of β-D-mannuronic acid and α-L-guluronic acid, oligomers thereof, or pharmaceutically acceptable salts thereof, wherein the total amount of β-D-mannuronic acid or α-L-guluronic acid, or their mixture, oligomers thereof, or pharmaceutically acceptable salts thereof is 10 wt.-% to 100 wt.-% based on the total weight of the tablet or capsule or 20 wt.-% to 60 wt.-% based on the total weight of the ampoule formulation. Additionally, the invention relates to tablet, capsule and ampoule formulations for oral and/or injectable use, preferably as a supplement or medication, in a method for treating neurodegeneration, inflammatory reactions, pain, cancer, aging, diabetes, and/or cadiopathy, and methods for preparing such formulations.

### BACKGROUND OF THE INVENTION

Mannuronic acid and guluronic acid are natural uronic acids which are isolated and purified from various forms of alginates, which are polymers used as a thickener, binder, gelling agent or lubricant. The source of alginate can be brown sea-weeds, e.g., *Phaeophyceae* and kelp, and/or bacteria belonging to the genera *Pseudomonas* and *Azotobacter.* The properties of the alginate vary, depending on which species it comes from. Alginates are mainly available in the form of sodium, calcium and ammonium salts. The difference in their monomer structures (mannuronic and guluronic acids) as well as the distribution, proportion and the length of these monomer blocks determine the chemical and physical properties of alginate polymers and oligomers.

Mannuronic and guluronic acids are epimers of each other. Therefore, many physicochemical and biological properties of these two molecules, e.g., molecular formula (C₆H₁₀O₇), molecular weight (194.14 g/mol), boiling point (553.4±50.0 °C at 760 mmHg), and density (1.7±0.1 g/cm³), are the same or highly similar.

Therapeutic properties of mannuronic acid in various animal models were first reported in 2004. The results of these studies revealed the therapeutic efficacy and tolerability of mannuronic acid in *in vitro* and *in vivo* experiments. The potent therapeutic efficacy of mannuronic acid was reported by Mirshafiey et al. 2005 (Mirshafiey A, Cuzzocrea S, Rehm BHA, H. Matsuo H. "M2000: a revolution in pharmacology", Med Sci Monit. 2005. 11(8):153-163). Later studies were carried out to address safety profile, the underlying cellular, molecular and immunological mechanisms and its therapeutic effects at the level of human clinical trials. The results of various studies and clinical trials confirmed that mannuronic acid is very safe and provides for a wide spectrum of therapeutic effects in a variety of diseases, such as rheumatoid arthritis, ankylosing spondylitis, breast cancer, multiple sclerosis, and myelodisplastic syndrome (Fattahi MJ, Jamshidi AR, Mahmoudi M, et al. "Evaluation of the efficacy and safety of β-d-mannuronic acid in patients with ankylosing spondylitis", "A 12-week randomized, placebo-controlled, phase I/II clinical trial", International Immunopharmacology. 2018; 54:112-117; Ahmadi H, Jamshidi AR, Gharibdoost F, et al, "A phase I/II randomized, controlled, clinical trial for assessment of the efficacy and safety of β-D-mannuronic acid in rheumatoid arthritis patients". Inflammopharmacology. 2018; 26(3):737-745; Rezaieyazdi Z, Farooqi A, Soleymani-Salehabadi H, et al. "International multicenter randomized, placebo-controlled phase III clinical trial of β-D-mannuronic acid in rheumatoid arthritis patients". Inflammopharmacology. 2019; 27(5):911-921; Kashefi S, Omranipour R, Mahmoodzadeh H, Ahmadi H, Mirshafiey A. "Clinical improvement of diabetes mellitus type 1 by β-D-mannuronic acid (M2000) in a breast cancer patient - as a case report", Clin Diabetol. 2019; 8(4):227-229; Najafi S, Moghadam NB, Saadat P, et al. "A controlled, randomized phase II clinical trial for efficacy and safety evaluation of mannuronic acid in secondary progressive form of multiple sclerosis", Int J Neurosci. 2022; 132(4):403-412; Ghaderi A, Nodehi SRS, Bakhtiari T, et al, "Mannuronic Acid in Low-Risk and Intermediate-1-Risk Myelodysplastic Syndromes". J Clin Pharmacol. 2020; 60(7):879-888).

The systematic pharmacology and medical investigations on guluronic acid were started in 2013 by Mirshafiey et al, and the results were published in 2015 ("New therapeutic approach by G2013 in experimental model of multiple sclerosis" Afraei S, Azizi G, Zargar SJ, Sedaghat R, Mirshafiey A., in; Acta Neurol Belg. 2015 Sep;115(3):259-66). Furthermore, the safety and pharmacotoxicology characteristics of guluronic acid as well as its clinical efficacy on inflammatory diseases have been studied.

These studies showed that not only share these two uronic acids many physicochemical properties, but also have the same or similar biological and medical properties.

### SUMMARY OF THE INVENTION

Surprisingly, it has been found that β-D-mannuronic acid or α-L-guluronic acid, oligomers thereof as well as the pharmaceutically acceptable salts thereof or mixtures of β-D-mannuronic acid and α-L-guluronic acid, oligomers thereof, or pharmaceutically acceptable salts thereof, preferably mannuronate or guluronate or gulumannuronate, can be effectively administered orally and/or as an injection in the form of a tablet, capsule or ampoule formulation, e.g. as an antioxidant.

Therefore, in a first aspect, the present invention relates to a tablet, capsule or ampoule formulation for oral and/or injectable use, the formulation comprising
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) a mixture of β-D-mannuronic acid, an oligomer thereof, or a pharmaceutically acceptable salt thereof and α-L-guluronic acid, an oligomer thereof, or a pharmaceutically acceptable salt thereof, preferably gulumannuronate,
wherein the total amount of β-D-mannuronic acid or α-L-guluronic acid, or their mixture, oligomers thereof, or pharmaceutically acceptable salts thereof is 10 wt.-% to 100 wt.-% based on the total weight of the tablet or capsule and/or 20 wt.-% to 60 wt.-% based on the total weight of the ampoule formulation.

In various embodiments, the tablet, capsule or ampoule formulation is a tablet or capsule formulation for oral and/or injectable use, preferably for oral use, the formulation comprising
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) a mixture of β-D-mannuronic acid, an oligomer thereof, or a pharmaceutically acceptable salt thereof and α-L-guluronic acid, an oligomer thereof, or a pharmaceutically acceptable salt thereof, preferably gulumannuronate,
wherein the total amount of β-D-mannuronic acid or α-L-guluronic acid, or their mixture, oligomers thereof, or pharmaceutically acceptable salts thereof is 10 wt.-% to 100 wt.-% based on the total weight of the tablet or capsule.

In various embodiments, the tablet, capsule or ampoule formulation is an ampoule formulation for oral and/or injectable use, preferably injectable use, the formulation comprising
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) a mixture of β-D-mannuronic acid, an oligomer thereof, or a pharmaceutically acceptable salt thereof and α-L-guluronic acid, an oligomer thereof, or a pharmaceutically acceptable salt thereof, preferably gulumannuronate,
wherein the total amount of β-D-mannuronic acid or α-L-guluronic acid, or their mixture, oligomers thereof, or pharmaceutically acceptable salts thereof is 20 wt.-% to 60 wt.-% based on the total weight of the ampoule formulation.

Preferably,
(i) the β-D-mannuronic acid, an oligomer thereof, or a pharmaceutically acceptable salt thereof is β-D-mannuronate, more preferably selected from the group consisting of sodium β-D-mannuronate, potassium β-D-mannuronate, magnesium β-D-mannuronate, calcium β-D-mannuronate or ammonium β-D-mannuronate and combinations thereof, more preferably sodium β-D-mannuronate; and/or
(ii) the α-L-guluronic acid, an oligomer thereof, or a pharmaceutically acceptable salt thereof is α-L-guluronate, more preferably selected from the group consisting of sodium α-L-guluronate, potassium α-L-guluronate, magnesium α-L-guluronate, calcium α-L-guluronate or ammonium α-L-guluronate and combinations thereof, more preferably sodium α-L-guluronate.

In embodiments where a mixture of β-D-mannuronic acid and α-L-guluronic acid, the respective oligomers thereof, or the respective pharmaceutically acceptable salts thereof is used, the active (pharmaceutical) agent may be gulumannuronate, preferably
wherein β-D-mannuronic acid and α-L-guluronic acid, oligomers thereof, or pharmaceutically acceptable salts thereof are mixed and preferably selected from the group consisting of sodium, or potassium, or magnesium, or calcium, or ammonium β-D-mannuronate and α-L-guluronate and combinations thereof, most preferably sodium β-D-mannuronate and/or sodium α-L-guluronate, or
wherein the mixture of β-D-mannuronic acid and α-L-guluronic acid, oligomers thereof, or pharmaceutically acceptable salts thereof, preferably gulumannuronate, is alginate hydrolysate (powder).

In various embodiments, the total amount of
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) the mixture of β-D-mannuronic acid and α-L-guluronic acid, oligomers thereof, or pharmaceutically acceptable salts thereof,

in the tablet or capsule is 30 wt.-% to 100 wt.-%, preferably 50 wt.-% to 100 wt.-%, based on the total weight of the tablet or capsule, and/or
in the ampoule formulation 30 wt.-% to 60 wt.-%, preferably 40 wt.-% to 60 wt.-%, more preferably 50 wt.-% to 60 wt.-%, based on the total weight of ampoule formulation.

In various embodiments, the tablet, capsule or ampoule formulation can further comprise at least one further supplement and/or pharmaceutical agent and/or at least one further ingredient as described herein.

Preferably, the tablet, capsule or ampoule formulation is a supplement and/or medication. In various embodiments, the tablet, capsule or ampoule formulation is for oral and/or injectable use as an antioxidant as described herein.

In a second aspect, the tablet, capsule or ampoule formulation is for oral and/or injectable use in a method for treating (or preventing)
(i) neurodegeneration, preferably in MS and Alzheimer's diseases; and/or
(ii) inflammatory reactions, preferably in rheumatic diseases; and/or
(iii) pain, preferably in joints and/or muscles; and/or
(iv) cancer, preferably in breast and prostate cancers; and/or
(v) aging; and/or
(vi) diabetes; and/or
(vii) cadiopathy; preferably in heart arrhythmia.

In a third aspect, the present invention relates to a method for preparing a tablet, capsule or ampoule formulation according to the inventions. Preferably, the tablet, capsule or ampoule formulation is an antioxidant tablet, capsule or ampoule formulation.

In various embodiments, the method is a method for preparing an antioxidant tablet, capsule or ampoule formulation, wherein the method comprises the steps of
providing a composition:
(a) comprising β-D-mannuronic acid, an oligomer thereof, or a pharmaceutically acceptable salt thereof, preferably β-D-mannuronate, in a total amount of 10 wt.-% to 100 wt.-% based on the total weight of the tablet or capsule and/or 20 wt.-% to 60 wt.-% based on the total weight of the ampoule formulation; or
(b) comprising α-L-guluronic acid, an oligomer thereof, or a pharmaceutically acceptable salt thereof, preferably α-L-guluronate, in a total amount of 10 wt.-% to 100 wt.-% based on the total weight of the tablet or capsule and/or 20 wt.-% to 60 wt.-% based on the total weight of the ampoule formulation; or
(c) comprising a mixture of β-D-mannuronic acid and α-L-guluronic acid, oligomers thereof, or pharmaceutically acceptable salts thereof, preferably gulumannuronic acid, gulumannuronate or oligomers thereof, in a total amount of 10 wt.-% to 100 wt.-% based on the total weight of the tablet or capsule and/or 20 wt.-% to 60 wt.-% based on the total weight of the ampoule formulation.

These and other aspects, embodiments, features, and advantages of the invention become apparent to the person skilled in the art in the following detailed description and claims. Each feature from one aspect of the invention can be used in any other aspect of the invention. Furthermore, the examples contained herein are intended to describe and illustrate the invention, but do not restrict it. In particular, the invention is not limited to these examples.

### DETAILED DESCRIPTION

"At least one", as used herein, means one or more, i.e. 1, 2, 3, 4, 5, 6, 7, 8, 9 or more of the referenced species. Similarly, "one or more", as used herein, relates to at least one and comprises 1, 2, 3, 4, 5, 6, 7, 8, 9 or more. In connection with a given species, the term does not relate to the total number of molecules, but rather to the type of species. "At least one preservative", for example, thus means that one type of preservative or two or more different types of preservatives may be present. In connection with amounts, the term relates to the total amount of the referenced species. In case of preservatives, for example, this means that the given amount is the total amount of all preservatives in the composition/formulation.

As used herein, unless otherwise stated, the singular forms "a", "an", and "the" include plural reference. Thus, for example, a reference to "an oligomer thereof' also includes a plurality of oligomer molecules or oligomer types, e.g. a mixture or combination of various oligomer types. Furthermore, unless otherwise stated, the use of plural forms, e.g. "oligomers thereof' or "pharmaceutical acceptable salts thereof", also includes singular reference such as one type of oligomer or one type of salt.

Numeric values specified without decimal places herein refer to the full value specified with one decimal place, i.e. for example, 99 % means 99.0 %, unless otherwise defined.

The term "about", in connection with a numerical value, refers to a variance of ±10 %, preferably ±5 %, with respect to the given numerical value.

The term "essentially free" within the context of this invention is to be interpreted as the respective compound is contained in the formulation in an amount of less than 5 wt.-%, 4 wt.-%, 3 wt.-%, 2 wt.-%, 1.5 wt.-%, 1 wt.-%, 0.75 wt.-%, 0.5 wt.-%, 0.25 wt.-%, 0.1 wt.-%, 0.01 wt.-%, or 0.001 wt.-% based on the total weight of the formulation, wherein the amounts are respectively more preferred in descending order. For example, 4 wt.-% is more preferred than 5 wt.-% and 3 wt.-% is more preferred than 4 wt.-%.

All percentages given herein in relation to the formulations relate to weight-% (wt.-%) relative to the total weight of the respective formulations, if not explicitly stated otherwise. Numeric ranges specified in the format "from x to y" include the specified values. If multiple preferred numeric ranges are specified in this format, it is understood that all ranges created by combining the different endpoints are also included.

In the following, the term "tablet, capsule or ampoule formulation" is used to describe the tablet, capsule or ampoule formulation for oral and/or injectable use, preferably as a supplement and/or medication, in particular as an antioxidant.

The terms "tablet (s)" and "tablet formulation(s)" as well as "capsule(s)" and "capsule formulation(s)" are intended to encompass all solid formulation types typically used in the field and also include lozenges, pills, film-coated tablets, compressettes and pellets. These are typically used for oral administration.

In various embodiments, tablets are, e.g., portioned powders, granules or substrates compressed under pressure, preferably compressed powders. They may have any suitable form and size, for example a biconvex form.

In various embodiments, capsules, such as hard and soft capsules, microcapsules or enteric-coated capsules, may contain solid fillings, such as powders, granulates, tablets, or even smaller capsules, or liquid or paste-like fillings, e.g., solutions, suspensions, or emulsions. Preferably, hard capsules contain solid fillings. The capsule coating may comprise or consist of gelatin, cellulose, carrageen, starch, glue or derivatives or combinations thereof, without being limited thereto.

Preferably, tablets and/or capsules are administered to a subject (in need thereof) orally. In various embodiments, tablets and/or capsules can be dissolved before oral use.

In the context of the present invention, an "ampoule formulation" is intended to encompass all injection formulation types typically used in the field, e.g., a liquid or powder formulation in an ampoule container or vessel, preferably for oral and/or injectable use, in particular injectable use. The material of the ampoule container or vessel may be glass or plastic, without being limited thereto. If the formulation is a powder formulation, the formulation can be dissolved, e.g. with water, before use. Ampoule formulations are typically used for injection purposes.

Tablet, capsule or ampoule formulation preparations are known to those skilled in the art and can be routinely prepared.

Beta-D-mannuronic acid has the following formula (I):

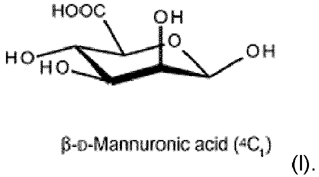

Alpha-L-guluronic acid has the following formula (II):

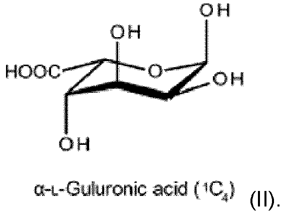

As mannuronic acid and guluronic acid are epimers, they share many physicochemical properties as well as biological and medicinal properties, for example as antioxidant agents. Therefore, in the present invention, all concepts disclosed for β-D-mannuronic acid, an oligomer thereof, or a pharmaceutically acceptable salt thereof are similarly applicable to α-L-guluronic acid, an oligomer thereof, or a pharmaceutically acceptable salt thereof, or to mixtures of both, and *vice versa.*

Free radicals may play a role in various diseases, e.g. in cancer, cardiovascular diseases, atherosclerosis, arthritis, aging, depression, anxiety and other diseases. On the one hand, these free radicals may be formed by the body itself during various metabolic processes, and on the other hand, they may be produced by harmful external influences such as cigarette smoke, environmental toxins or UV radiation from the sun. Antioxidants are, typically, substances that inhibit and/or reduce oxidation, a chemical reaction that can produce free radicals and chain reactions that may damage the cells of organisms. Therefore, antioxidants may act as prophylactic and therapeutic agents.

According to the invention, β-D-mannuronic acid or α-L-guluronic acid or their mixtures can be comprised in the tablet, capsule or ampoule formulation in form of pharmaceutically acceptable salts. Preferably, these pharmaceutically acceptable salts are mannuronate, guluronate, or a mixture thereof, more preferably β-D-mannuronate, or α-L-guluronate, or a mixture thereof referred to as "gulumannuronate". The counterion may be selected from sodium, potassium, magnesium, calcium, ammonium and other pharmaceutically acceptable cationic counterions.

Thus, in various embodiments, the tablet, capsule or ampoule formulation for oral and/or injectable use can be:
(i) a mannuronate tablet, capsule or ampoule formulation; or
(ii) a guluronate tablet, capsule or ampoule formulation; or
(iii) a gulumannuronate tablet, capsule or ampoule formulation.

In various embodiments, the tablet, capsule or ampoule formulation comprising β-D-mannuronic acid, an oligomer thereof, or a pharmaceutically acceptable salt thereof, is a mannuronate tablet, capsule or ampoule formulation. Specifically, the β-D-mannuronic acid, an oligomer thereof, or a pharmaceutically acceptable salt thereof can be, in various embodiments, β-D-mannuronate, more preferably selected from the group consisting of sodium β-D-mannuronate, potassium β-D-mannuronate, magnesium β-D-mannuronate, calcium β-D-mannuronate, ammonium β-D-mannuronate and combinations thereof, with sodium β-D-mannuronate being especially preferred.

In various embodiments, β-D-mannuronic acid, preferably β-D-mannuronate, can also be added to the tablet, capsule or ampoule formulation in the form of a precursor substance selected from oligomers of β-D-mannuronic acid or β-D-mannuronate, preferably (homo)oligomers of β-D-mannuronate, in particular sodium oligomannuronate, potassium oligomannuronate, magnesium oligomannuronate, calcium oligomannuronate, ammonium oligomannuronate and combinations thereof, especially sodium oligomannuronate.

In various embodiments, β-D-mannuronic acid, an oligomer thereof, or a pharmaceutically acceptable salt thereof, preferably β-D-mannuronate, is selected from the group consisting of sodium β-D-mannuronate, potassium β-D-mannuronate, magnesium β-D-mannuronate, calcium β-D-mannuronate, ammonium β-D-mannuronate, sodium oligomannuronate, potassium oligomannuronate, magnesium oligomannuronate, calcium oligomannuronate, ammonium oligomannuronate and combinations thereof, preferably sodium β-D-mannuronate and/or sodium oligomannuronate.

In various embodiments, the tablet, capsule or ampoule formulation comprising α-L-guluronic acid, an oligomer thereof, or a pharmaceutically acceptable salt thereof is a guluronate tablet, capsule or ampoule formulation. Specifically, the α-L-guluronic acid, an oligomer thereof, or a pharmaceutically acceptable salt thereof can be, in various embodiments, α-L-guluronate, preferably selected from the group consisting of sodium α-L-guluronate, potassium α-L-guluronate, magnesium α-L-guluronate, calcium α-L-guluronate, ammonium α-L-guluronate and combinations thereof, with sodium α-L-guluronate being especially preferred.

In various embodiments, α-L-guluronic acid, preferably α-L-guluronate, can also be added to the tablet, capsule or ampoule formulation in the form of a precursor substance selected from oligomers of α-L-guluronic acid or α-L-guluronate, preferably (homo)oligomers of α-L-guluronate, in particular sodium oligoguluronate, potassium oligoguluronate, magnesium oligoguluronate, calcium oligoguluronate, ammonium oligoguluronate and combinations thereof, especially sodium oligoguluronate.

In various embodiments, α-L-guluronic acid, an oligomer thereof, or a pharmaceutically acceptable salt thereof, preferably α-L-guluronate, is selected from the group consisting of sodium α-L-guluronate, potassium α-L-guluronate, magnesium α-L-guluronate, calcium α-L-guluronate, ammonium α-L-guluronate, sodium oligoguluronate, potassium oligoguluronate, magnesium oligoguluronate, calcium oligoguluronate, ammonium oligoguluronate and combinations thereof, preferably sodium α-L-guluronate and/or sodium oligoguluronate.

In various embodiments, the tablet, capsule or ampoule formulation comprising a mixture of β-D-mannuronic acid and α-L-guluronic acid, oligomers thereof, or pharmaceutically acceptable salts thereof, is a gulumannuronate tablet, capsule or ampoule formulation. In particular, the mixture of β-D-mannuronic acid and α-L-guluronic acid, oligomers thereof, or pharmaceutically acceptable salts thereof can be a mixture of β-D-mannuronate and α-L-guluronate, preferably selected from the group consisting of sodium β-D-mannuronate, potassium β-D-mannuronate, magnesium β-D-mannuronate, calcium β-D-mannuronate or ammonium β-D-mannuronate or combinations thereof and sodium α-L-guluronate, potassium α-L-guluronate, magnesium α-L-guluronate, calcium α-L-guluronate or ammonium α-L-guluronate or combinations thereof, with a mixture of sodium β-D-mannuronate and sodium α-L-guluronate being especially preferred.

In various embodiments, the mixture of β-D-mannuronic acid and α-L-guluronic acid, preferably gulumannuronate, can also be added to the tablet, capsule or ampoule formulation in the form of precursor substances selected from oligomers of β-D-mannuronic acid/β-D-mannuronate and/or α-L-guluronic acid/α-L-guluronate, preferably (homo)oligomers of β-D-mannuronic acid/β-D-mannuronate and/or α-L-guluronic acid/α-L-guluronate, in particular sodium oligomannuronate, potassium oligomannuronate, magnesium oligomannuronate, calcium oligomannuronate, ammonium oligomannuronate, sodium oligoguluronate, potassium oligoguluronate, magnesium oligoguluronate, calcium oligoguluronate, ammonium oligoguluronate and combinations thereof, especially sodium oligomannuronate and/or sodium oligoguluronate.

In various embodiments, β-D-mannuronic acid and/or α-L-guluronic acid, oligomers thereof, or pharmaceutically acceptable salts thereof, preferably gulumannuronate, is selected from the group consisting of sodium β-D-mannuronate, potassium β-D-mannuronate, magnesium β-D-mannuronate, calcium β-D-mannuronate, ammonium β-D-mannuronate, sodium oligomannuronate, potassium oligomannuronate, magnesium oligomannuronate, calcium oligomannuronate, ammonium oligomannuronate, sodium α-L-guluronate, potassium α-L-guluronate, magnesium α-L-guluronate, calcium α-L-guluronate, ammonium α-L-guluronate, sodium oligoguluronate, potassium oligoguluronate, magnesium oligoguluronate, calcium oligoguluronate, ammonium oligoguluronate and combinations thereof, preferably sodium β-D-mannuronate, sodium oligomannuronate, sodium α-L-guluronate, sodium oligoguluronate and combinations thereof.

In particular, β-D-mannuronate oligomers or α-L-guluronate oligomers comprise 2 to 16 monomeric units, preferably 3 to 6 monomeric units, e.g., 3 or 4 or 5 or 6 monomeric units.

In various embodiments, the mixture of β-D-mannuronic acid and α-L-guluronic acid, oligomers thereof, or pharmaceutically acceptable salts thereof, preferably gulumannuronate, can be synthesized from sodium alginate and can be used in the tablet, capsule or ampoule formulation in the form of alginate hydrolysate, preferably alginate hydrolysate powder, which is also referred to as gulumannuronate. In particular, alginate hydrolysate (gulumannuronate) is the separated precipitate of sodium alginate comprising mannuronic acid/mannuronate and guluronic acid/guluronate. Therefore, in various embodiments, the tablet, capsule or ampoule formulation comprises or consists of alginate hydrolysate (gulumannuronate), preferably
in the tablet or capsule in amounts of 10 wt.-% to 100 wt.-%, more preferably 30 wt.-% to 100 wt.-%, more preferably 40 wt.-% to 100 wt.-%, more preferably 50 wt.-% to 100 wt.-%, based on the total weight of the tablet or capsule, and/or
in the ampoule formulation in amounts of 20 wt.-% to 60 wt.-%, more preferably 30 wt.-% to 60 wt.-%, more preferably 40 wt.-% to 60 wt.-%, more preferably 50 wt.-% to 60 wt.-%, based on the total weight of ampoule formulation.

It is preferred that the mixture of β-D-mannuronic acid and α-L-guluronic acid, oligomers thereof, or pharmaceutically acceptable salts thereof, preferably gulumannuronate, may be (1) a mixture of the single components β-D-mannuronic acid, oligomers thereof, or pharmaceutically acceptable salts thereof, in particular β-D-mannuronate (powder) and α-L-guluronic acid, oligomers thereof, or pharmaceutically acceptable salts thereof, in particular α-L-guluronate (powder); or (2) alginate hydrolysate (powder).

In various embodiments, the total amount of
(i) β-D-mannuronic acid, an oligomer thereof or a pharmaceutically acceptable salt thereof, or
(ii) α-L-guluronic acid, an oligomer thereof, or a pharmaceutically acceptable salt thereof, or
(iii) the mixture of β-D-mannuronic acid and α-L-guluronic acid, oligomers thereof, or pharmaceutically acceptable salts thereof,

in the tablet or capsule formulation is 20 wt.-% to 100 wt.-%, preferably 30 wt.-% to 100 wt.-%, more preferably 40 wt.-% to 100 wt.-%, more preferably 50 wt.-% to 100 wt.-%, based on the total weight of the tablet or capsule, and/or
in the ampoule formulation is 30 wt.-% to 60 wt.-%, preferably 40 wt.-% to 60 wt.-%, more preferably 50 wt.-% to 60 wt.-%, e.g., 50 wt.-% or 55 wt.-%, based on the total weight of ampoule formulation.

In various embodiments, the tablet or capsule, preferably the tablet consists of
(i) β-D-mannuronic acid, an oligomer thereof or a pharmaceutically acceptable salt thereof, or
(ii) α-L-guluronic acid, an oligomer thereof, or a pharmaceutically acceptable salt thereof, or
(iii) the mixture of β-D-mannuronic acid and α-L-guluronic acid, oligomers thereof, or pharmaceutically acceptable salts thereof. In this case, the tablet or capsule formulation is free or essentially free of further supplements, pharmaceutical agents and/or ingredients.

In one embodiment, the total amount of β-D-mannuronic acid, an oligomer thereof, or a pharmaceutically acceptable salt thereof in the tablet or capsule formulation, preferably in the mannuronate tablet or capsule formulation, is 20 wt.-% to 100 wt.-%, preferably 30 wt.-% to 100 wt.-%, more preferably 40 wt.-% to 100 wt.-%, more preferably 50 wt.-% to 100 wt.-%, based on the total weight of the tablet or capsule; or in the ampoule formulation, preferably in the mannuronate ampoule formulation is 30 wt.-% to 60 wt.-%, preferably 40 wt.-% to 60 wt.-%, more preferably 50 wt.-% to 60 wt.-%, e.g., 50 wt.-% or 55 wt.-%, based on the total weight of ampoule formulation.

In another embodiment, the total amount of α-L-guluronic acid, an oligomer thereof, or a pharmaceutically acceptable salt thereof in the tablet or capsule formulation, preferably in the guluronate tablet or capsule formulation, is 20 wt.-% to 100 wt.-%, preferably 30 wt.-% to 100 wt.-%, more preferably 40 wt.-% to 100 wt.-%, more preferably 50 wt.-% to 100 wt.-%, based on the total weight of the tablet or capsule; or in the ampoule formulation, preferably in the guluronate ampoule formulation is 30 wt.-% to 60 wt.-%, preferably 40 wt.-% to 60 wt.-%, more preferably 50 wt.-% to 60 wt.-%, e.g., 50 wt.-% or 55 wt.-%, based on the total weight of ampoule formulation.

In another embodiment, the total amount of the mixture of β-D-mannuronic acid and α-L-guluronic acid, oligomers thereof, or pharmaceutically acceptable salts thereof in the tablet or capsule formulation, preferably in the gulumannuronate tablet or capsule formulation, is 20 wt.-% to 100 wt.-%, preferably 30 wt.-% to 100 wt.-%, more preferably 40 wt.-% to 100 wt.-%, more preferably 50 wt.-% to 100 wt.-%, based on the total weight of the tablet or capsule; or in the ampoule formulation, preferably in the gulumannuronate ampoule formulation is 30 wt.-% to 60 wt.-%, preferably 40 wt.-% to 60 wt.-%, more preferably 50 wt.-% to 60 wt.-%, e.g., 50 wt.-% or 55 wt.-%, based on the total weight of ampoule formulation.

In various embodiments, the mixture of β-D-mannuronic acid and α-L-guluronic acid, oligomers thereof, or pharmaceutically acceptable salts thereof, preferably (in) the gulumannuronate tablet or capsule formulation, preferably comprises or consists of 0.01 wt.-% to 99.99 wt.-% of β-D-mannuronic acid, an oligomer thereof, or a pharmaceutically acceptable salt thereof, and 0.01 wt.-% to 99.99 wt.-% of α-L-guluronic acid, an oligomer thereof, or a pharmaceutically acceptable salt thereof, with the proviso that the total amount of β-D-mannuronic acid, an oligomer thereof, or a pharmaceutically acceptable salt thereof and α-L-guluronic acid, an oligomer thereof, or a pharmaceutically acceptable salt thereof in the tablet or capsule formulation is at least 10 wt.-% and at most 100 wt.-%, based on the total weight of the tablet or capsule, preferably
the total amount of β-D-mannuronic acid, an oligomer thereof, or a pharmaceutically acceptable salt thereof is 0.1 wt.-% to 99.9 wt.-%, more preferably 1 wt.-% to 99 wt.-%, more preferably 10 wt.-% to 90 wt.-%, based on the total weight of the tablet or capsule formulation, and
the total amount of α-L-guluronic acid, an oligomer thereof, or a pharmaceutically acceptable salt thereof is 0.1 wt.-% to 99.9 wt.-%, preferably 1 wt.-% to 99 wt.-%, more preferably 10 wt.-% to 90 wt.-%, based on the total weight of the tablet or capsule formulation. In various embodiments, the weight ratio of β-D-mannuronic acid, an oligomer thereof, or a pharmaceutically acceptable salt thereof to α-L-guluronic acid, an oligomer thereof, or a pharmaceutically acceptable salt thereof is in the range of from 100:1 to 1:100, such as 10:1 to 1:10 or 5:1 to 1:5, preferably 3:1 to 1:3, 2:1 to 1:2 or about 1.5:1 to 1:1.5, most preferably about 1:1.

In various embodiments, the mixture of β-D-mannuronic acid and α-L-guluronic acid, oligomers thereof, or pharmaceutically acceptable salts thereof, preferably (in) the gulumannuronate ampoule formulation, preferably comprises or consists of 0.01 wt.-% to 59.99 wt.-% of β-D-mannuronic acid, an oligomer thereof, or a pharmaceutically acceptable salt thereof, and 0.01 wt.-% to 59.99 wt.-% of α-L-guluronic acid, an oligomer thereof, or a pharmaceutically acceptable salt thereof, with the proviso that the total amount of β-D-mannuronic acid, an oligomer thereof, or a pharmaceutically acceptable salt thereof and α-L-guluronic acid, an oligomer thereof, or a pharmaceutically acceptable salt thereof in the ampoule formulation is at least 20 wt.-% and at most 60 wt.-%, based on the total weight of the ampoule formulation, preferably
the total amount of β-D-mannuronic acid, an oligomer thereof, or a pharmaceutically acceptable salt thereof is 0.1 wt.-% to 59.9 wt.-%, more preferably 1 wt.-% to 59 wt.-%, more preferably 10 wt.-% to 50 wt.-%, more preferably 20 wt.-% to 40 wt.-%, based on the total weight of the ampoule formulation, and the total amount of α-L-guluronic acid, an oligomer thereof, or a pharmaceutically acceptable salt thereof is 0.1 wt.-% to 59.9 wt.-%, more preferably 1 wt.-% to 59 wt.-%, more preferably 10 wt.-% to 50 wt.-%, more preferably 20 wt.-% to 40 wt.-%, based on the total weight of ampoule formulation. In various embodiments, the weight ratio of β-D-mannuronic acid, an oligomer thereof, or a pharmaceutically acceptable salt thereof to α-L-guluronic acid, an oligomer thereof, or a pharmaceutically acceptable salt thereof is in the range of from 100:1 to 1:100, such as 10:1 to 1:10 or 5:1 to 1:5, preferably 3:1 to 1:3, 2:1 to 1:2 or about 1.5:1 to 1:1.5, most preferably about 1:1.

In various embodiments, the tablet, capsule or ampoule formulation according to the invention can comprise β-D-mannuronic acid or α-L-guluronic acid, oligomers thereof, or pharmaceutically acceptable salts thereof, or their mixture, preferably β-D-mannuronate or α-L-guluronate or their mixture, together with other, preferably chemically pure, supplements, pharmaceutical agents, drugs and/or herbal medicaments.

In various embodiments, the tablet, capsule or ampoule formulation comprises at least one further supplement and/or pharmaceutical agent.

Supplements are, in particular, nutrients, that can be added to a drug or formulation according to the invention. In various embodiments, supplements are intended to supplement the diet of a subject in order to supply the body optimally.

Pharmaceutical agents are, preferably, the active components of a drug or formulation that are responsible for its pharmacological effects.

In various embodiments, without being limited thereto, the at least one further supplement and/or at least one further pharmaceutical agent, in particular for tablet or capsule formulations, is selected from antioxidant agents, anticancer agents, vitamins, multivitamins, (pharmaceutical) minerals, (pharmaceutical) multiminerals, amino acids, fatty acids, herbs, carbohydrates, analgesic agents, anti-inflammatory agents and agents with anti-sensitivity properties or combinations thereof, without being limited thereto.

Suitable further antioxidant agents (from foods) are, for example, beta-carotene, OPCs, resveratrol, flavonoids, lycopene, anthocyanins, zeaxanthins, chlorophyll and/or allicin, without being limited thereto. Further suitable antioxidant agents can be thiols.

Vitamins that can be used as further supplements and/or pharmaceutical agents are, e.g., vitamin A, C, D, E or K(2), without being limited thereto.

Anticancer agents are, for example, curcumin, taxol or selenium, without being limited thereto.

Suitable pharmaceutical minerals are, for example, calcium carbonate, calcium citrate, calcium malate, magnesium glycinate, magnesium citrate, magnesium gluconate, magnesium lactate, phosphorus-based substances, sodium chloride and/or potassium chloride, without being limited thereto.

An analgesic agent is defined, in the context of the present invention, as a drug that eliminates, alleviates or relieves pain. Examples are, without being limited thereto, methocarbamol, baclofen, non-steroidal anti-inflammatory drugs (NSAIDs) and/or opioids.

A suitable anti-inflammatory agent according to the invention is a drug that reduces or eliminates inflammation or inflammatory reaction in the body, e.g., non-steroidal anti-inflammatory drugs (NSAIDs) such as COX-2 inhibitors (coxibs) diclofenac, naproxen or ibuprofen, and/or corticosteroids such as cortisol, cortisone, prednisone and prednisolone, without being limited thereto.

Agents with anti-sensitivity properties, which can be added in particular to the tablet or capsule formulation for oral use, are all anti-allergy or anti-sensitive (desensitizing) agents safe for use in humans. Examples are, without being limited thereto, antihistamines and diphenhydramine HCI.

In preferred embodiments, the tablet, capsule or ampoule formulation is used orally and/or injectably as a supplement and/or as a medication.

In various embodiments, the tablet, capsule or ampoule formulation for oral and/or injectable use is an antioxidant formulation/supplement/drug, an anticancer formulation/supplement/drug, a vitamin formulation/supplement/drug, an analgesic formulation/supplement/drug, an anti-inflammatory formulation/supplement/drug and/or a formulation/supplement/drug with anti-sensitivity properties.

In various embodiments, the tablet, capsule or ampoule formulation, in particular the tablet or capsule formulation, further comprises caffeic acid or a salt thereof, for example in powder form, preferably in an amount of 0.001 wt.-% to 1 wt.-%, more preferably 0.01 wt.-% to 0.5 wt.-%, more preferably 0.05 wt.-% to 0.3 wt.-%, e.g., 0.1 wt.-% to 0.3 wt.-%, based on the total weight of the tablet, capsule or ampoule formulation, preferably based on the total weight of the tablet or capsule formulation.

Caffeic acid (3,4-dihydroxycinnamic acid, with the molecular formula C₉H₈O₄) may be found in many plants and foods. For example, coffee is the primary source of caffeic acid in the human diet. However, it can also be found in other food sources such as apples, pears, artichoke and berries. In various embodiments, caffeic acid may be used in medication and/or supplements for boosting athletic performance, exercise-related fatigue and weight loss, in particular it has many effects in the body including antioxidant, anti-cancer, anti-inflammatory, neuroprotective and photoprotective effects. Caffeic acid is commercially available, for example from Sigma Aldrich, or can be isolated from coffee or other food sources.

Therefore, in preferred embodiments, the tablet, capsule or ampoule formulation, preferably the tablet or capsule formulation, comprises
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) a mixture of β-D-mannuronic acid, an oligomer thereof, or a pharmaceutically acceptable salt thereof and α-L-guluronic acid, an oligomer thereof, or a pharmaceutically acceptable salt thereof, preferably gulumannuronate,
wherein the total amount of β-D-mannuronic acid or α-L-guluronic acid, or their mixture, oligomers thereof, or pharmaceutically acceptable salts thereof is 10 wt.-% to 100 wt.-% based on the total weight of the tablet or capsule and/or 20 wt.-% to 60 wt.-% based on the total weight of the ampoule formulation; in combination with caffeic acid or a salt thereof, for example in powder form, preferably in amounts of 0.001 wt.-% to 1 wt.-%, more preferably 0.01 wt.-% to 0.5 wt.-%, more preferably 0.05 wt.-% to 0.3 wt.-%, e.g., 0.1 wt.-% to 0.3 wt.-%, based on the total weight of the tablet, capsule or ampoule formulation, in particular based on the tablet or capsule formulation. It is preferred that such a formulation may be used as antioxidant medication or supplement.

In various embodiments, the tablet, capsule or ampoule formulation for oral and/or injectable use may contain further ingredients such as (one or more) auxiliaries known to those skilled in the art and conventionally used in such formulations.

For example, the following auxiliaries may be used in the tablet, capsule or ampoule formulations of the invention, in particular in the tablet or capsule formulations, without being limited thereto: particulate carriers (e.g., zinc oxide, starch, starch derivatives); antioxidants (e.g., allicin, beta-carotene, flavonoids, vitamin E, ascorbic acid and its derivatives); fillers (e.g., microcrystalline cellulose, sorbitol); sweeteners (e.g., aspartame, acesulfame K, sucralose, saccharin, neotame, advantame, alitame, dextrose, fructose, galactose, inulin, isomalt, sorbitol).

In various embodiments, the at least one further ingredient is selected from binders, fillers, builders, complexing agents, preservatives, coating agents, alkaline agents, acidifiers, sweeteners, colorants, buffers, acids and bases or combinations thereof.

According to the invention, the tablet, capsule or ampoule formulation is administered orally and/or injectably, preferably to a subject in need thereof.

In preferred embodiments, the subject is a mammalian, in particular a human.

In preferred embodiments, the tablet, capsule or ampoule formulation according to the invention is for oral and/or injectable use in a method for treating (or preventing)
(i) neurodegeneration, preferably in MS and Alzheimer's diseases; and/or
(ii) inflammatory reactions, preferably in rheumatic diseases; and/or
(iii) pain, preferably in joints and/or muscles; and/or
(iv) cancer, preferably in breast and/or prostate cancers; and/or
(v) aging; and/or
(vi) diabetes; and/or
(vii) cadiopathy, preferably in heart arrhythmia.

The term "neurodegeneration" may comprise all pathological processes that lead to a loss of function and/or the demise of nerve cells. Neurodegenerative diseases include various forms of Multiple sclerosis, Alzheimer's disease, or Amyotrophic lateral sclerosis (ALS) disorder, without being limited thereto. In particular, neurodegeneration comprises MS and Alzheimer's disease.

The tablet, capsule or ampoule formulation for oral and/or injectable use can be used to treat or prevent any type of "cancer". A "cancer" as used herein preferably includes cancer, particularly those of epithelial origin, which are characterized by abnormal cellular proliferation, which may manifest by tumor formation. The term encompasses cancer as that which is localized in tumors, as well as that which is not localized in tumors, such as cancer cells that undergo local inverse expansion from a tumor. In various embodiments, the present invention is applicable as a general (systemic) and/or local control agent for tumor growth, such as carcinomas of the bladder, breast, colon, kidney, liver, lung, ovary, pancreas, rectum and stomach, as well as a treatment for a sarcoma, e.g., fibrosarcoma or rhabdosarcoma, a hematopoietic tumor of the lymphoid or myeloid lineage, or other tumors, including but not limited to melanoma, teratocarcinoma, neuroblastoma, or glioma.

In preferred embodiments, the cancer includes leukemias, seminomas, melanomas, teratomas, gliomas, and/or colon cancer, rectal cancer, colorectal cancer, gastrointestinal cancer, esophageal cancer, throat cancer, nasal cancer, ear (ENT) cancer, kidney cancer, adrenal cancer, thyroid cancer, lymph node cancer, breast cancer, prostate cancer, uterine cancer, ovarian cancer, endometrium cancer, liver cancer, pancreas cancer, skin cancer, brain cancer, and lung cancer and their metastases, without being limited thereto, preferably breast and/or prostate cancers.

The term "aging" in the context of the present invention refers to any type of visible and invisible aging phenomena of the body of a subject. In various embodiments, the tablet, capsule or ampoule formulation for oral and/or injectable use is for treating, preventing or reducing wrinkles, fine lines and sagging skin and/or, e.g., to supply the body with suitable nutrients to treat, prevent or reduce aging phenomena and processes of the body or body cells.

"Cadiopathy" in the context of the present invention may refer to any disease or disorder of the heart, preferably heart arrhythmia.

In various embodiments, the tablet, capsule or ampoule formulation is an antioxidant tablet, capsule or ampoule formulation.

In further preferred embodiments, the (antioxidant) tablet, capsule or ampoule formulation according to the invention is orally and/or injectably administered to a subject in need thereof in a method for treating (or preventing)
(i) neurodegeneration, preferably in MS and Alzheimer's diseases; and/or
(ii) inflammatory reactions, preferably in rheumatic diseases; and/or
(iii) pain, preferably in joints and/or muscles; and/or
(iv) cancer, preferably in breast and/or prostate cancers; and/or
(v) aging; and/or
(vi) diabetes; and/or
(vii) cadiopathy, preferably in heart arrhythmia.

In such an embodiment, it may be preferred that the tablet, capsule or ampoule formulation comprises at least one further supplement and/or pharmaceutical agent as described above, preferably at least one antioxidant agents, anticancer agents, vitamins, multivitamins, (pharmaceutical) minerals, (pharmaceutical) multiminerals, amino acids, fatty acids, herbs, carbohydrates, analgesic agents, anti-inflammatory agents and agents with anti-sensitivity properties or combinations thereof, without being limited thereto.

In a further aspect, the present invention relates to a method for preparing a tablet, capsule or ampoule formulation according to the invention. Preferably, the tablet, capsule or ampoule formulation is a tablet, capsule or ampoule formulation for oral and/or injectable use as described herein.

According to the invention, the resulting tablet, capsule or ampoule formulation obtained by the method according to the invention comprises
(i) β-D-mannuronic acid, an oligomer thereof, or a pharmaceutically acceptable salt thereof, preferably β-D-mannuronate, in a total amount of 10 wt.-% to 100 wt.-% based on the total weight of the tablet or capsule, and/or 20 wt.-% to 60 wt.-% based on the total weight of the ampoule formulation; or
(ii) α-L-guluronic acid, an oligomer thereof, or a pharmaceutically acceptable salt thereof, preferably α-L-guluronate, in a total amount of 10 wt.-% to 100 wt.-% based on the total weight of the tablet or capsule, and/or 20 wt.-% to 60 wt.-% based on the total weight of the ampoule formulation; or
(iii) a mixture of β-D-mannuronic acid and α-L-guluronic acid, oligomers thereof, or pharmaceutically acceptable salts thereof, preferably gulumannuronic acid, gulumannuronate or oligomers thereof, in a total amount of 10 wt.-% to 100 wt.-% based on the total weight of the tablet or capsule, and/or 20 wt.-% to 60 wt.-% based on the total weight of the ampoule formulation.

Preferably, the tablet, capsule or ampoule formulation according to the invention comprises
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) the mixture of β-D-mannuronic acid, an oligomer thereof, or a pharmaceutically acceptable salt thereof and α-L-guluronic acid, an oligomer thereof, or a pharmaceutically acceptable salt thereof,

in an amount of 30 wt.-% to 100 wt.-%, more preferably 50 wt.-% to 100 wt.-%, based on the total weight of the tablet or capsule, and/or
in an amount of 30 wt.-% to 60 wt.-%, more preferably 40 wt.-% to 60 wt.-%, more preferably 50 wt.-% to 60 wt.-%, based on the total weight of the ampoule formulation.

In various embodiments, β-D-mannuronic acid, an oligomer thereof, or a pharmaceutically acceptable salt thereof, preferably β-D-mannuronate, and/or α-L-guluronic acid, an oligomer thereof, or a pharmaceutically acceptable salt thereof, preferably α-L-guluronate, is added in the method according to the invention in powder form.

In embodiments in which the resulting tablet, capsule or ampoule formulation is a gulumannuronate tablet, capsule or ampoule formulation, the tablet or capsule formulation preferably comprises or consists of 0.01 wt.-% to 99.99 wt.-% of β-D-mannuronic acid, an oligomer thereof, or a pharmaceutically acceptable salt thereof, and 0.01 wt.-% to 99.99 wt.-% of α-L-guluronic acid, an oligomer thereof, or a pharmaceutically acceptable salt thereof, with the proviso that the total amount of β-D-mannuronic acid, an oligomer thereof, or a pharmaceutically acceptable salt thereof and α-L-guluronic acid, an oligomer thereof, or a pharmaceutically acceptable salt thereof in the tablet or capsule formulation is at least 10 wt.-% and at most 100 wt.-%, based on the total weight of the tablet or capsule, preferably the total amount of β-D-mannuronic acid, an oligomer thereof, or a pharmaceutically acceptable salt thereof is 0.1 wt.-% to 99.9 wt.-%, more preferably 1 wt.-% to 99 wt.-%, more preferably 10 wt.-% to 90 wt.-%, based on the total weight of the tablet or capsule formulation, and the total amount of α-L-guluronic acid, an oligomer thereof, or a pharmaceutically acceptable salt thereof is 0.1 wt.-% to 99.9 wt.-%, preferably 1 wt.-% to 99 wt.-%, more preferably 10 wt.-% to 90 wt.-%, based on the total weight of the tablet or capsule formulation.

In various embodiments, in which the resulting tablet, capsule or ampoule formulation is a gulumannuronate tablet, capsule or ampoule formulation, the ampoule formulation preferably comprises or consists of 0.01 wt.-% to 59.99 wt.-% of β-D-mannuronic acid, an oligomer thereof, or a pharmaceutically acceptable salt thereof, and 0.01 wt.-% to 59.99 wt.-% of α-L-guluronic acid, an oligomer thereof, or a pharmaceutically acceptable salt thereof, with the proviso that the total amount of β-D-mannuronic acid, an oligomer thereof, or a pharmaceutically acceptable salt thereof and α-L-guluronic acid, an oligomer thereof, or a pharmaceutically acceptable salt thereof in the ampoule formulation is at least 20 wt.-% and at most 60 wt.-%, based on the total weight of the ampoule formulation, preferably
the total amount of β-D-mannuronic acid, an oligomer thereof, or a pharmaceutically acceptable salt thereof is 0.1 wt.-% to 59.9 wt.-%, more preferably 1 wt.-% to 59 wt.-%, more preferably 10 wt.-% to 50 wt.-%, more preferably 20 wt.-% to 40 wt.-%, based on the total weight of the ampoule formulation, and the total amount of α-L-guluronic acid, an oligomer thereof, or a pharmaceutically acceptable salt thereof is 0.1 wt.-% to 59.9 wt.-%, preferably 1 wt.-% to 59 wt.-%, more preferably 10 wt.-% to 50 wt.-%, more preferably 20 wt.-% to 40 wt.-%, based on the total weight of ampoule formulation.

In various embodiments, alginate hydrolysate (powder) can be used (as the mixture of β-D-mannuronic acid, an oligomer thereof, or a pharmaceutically acceptable salt thereof and α-L-guluronic acid, an oligomer thereof, or a pharmaceutically acceptable salt thereof) for gulumannuronate tablet, capsule or ampoule preparation. Preferably, alginate hydrolysate (powder) is used in the tablet or capsule formulation in total amounts of 30 wt.-% to 100 wt.-%, more preferably 40 wt.-% to 100 wt.-%, more preferably 50 wt.-% to 100 wt.-%, based on the total weight of the tablet or capsule, and/or
in the ampoule formulation in total amounts of 30 wt.-% to 60 wt.-%, preferably 40 wt.-% to 60 wt.-%, more preferably 50 wt.-% to 60 wt.-%, based on the total weight of the ampoule formulation.

In various embodiments, β-D-mannuronic acid, an oligomer thereof, or a pharmaceutically acceptable salt thereof, preferably β-D-mannuronate, is selected from the group consisting of sodium β-D-mannuronate, potassium β-D-mannuronate, magnesium β-D-mannuronate, calcium β-D-mannuronate, ammonium β-D-mannuronate, and combinations thereof, and/or sodium oligomannuronate, potassium oligomannuronate, magnesium oligomannuronate, calcium oligomannuronate, ammonium oligomannuronate and combinations thereof, with sodium β-D-mannuronate and/or sodium oligomannuronate being especially preferred in the mannuronate tablet, capsule or ampoule formulation preparation.

In various embodiments, α-L-guluronic acid, an oligomer thereof, or a pharmaceutically acceptable salt thereof, preferably α-L-guluronate, is selected from the group consisting of sodium α-L-guluronate, potassium α-L-guluronate, magnesium α-L-guluronate, calcium α-L-guluronate, ammonium α-L-guluronate, and combinations thereof, and/or sodium oligoguluronate, potassium oligoguluronate, magnesium oligoguluronate, calcium oligoguluronate, ammonium oligoguluronate and combinations thereof, with sodium α-L-guluronate and/or sodium oligoguluronate being especially preferred in the guluronate tablet, capsule or ampoule formulation preparation.

In various embodiments, the mixture of β-D-mannuronic acid and α-L-guluronic acid, oligomers thereof, or pharmaceutically acceptable salts thereof, preferably β-D-mannuronate and α-L-guluronate, are selected from the group consisting of sodium or potassium or magnesium or calcium or ammonium β-D-mannuronate and sodium or potassium or magnesium or calcium or ammonium α-L-guluronate and combinations thereof, and/or their oligomers, preferably their homooligomers consisting of sodium or potassium or magnesium or calcium or ammonium oligomannuronate and sodium or potassium or magnesium or calcium or ammonium oligoguluronate and combinations thereof, with sodium β-D-mannuronate, sodium α-L-guluronate, sodium oligomannuronate and sodium oligoguluronate, and combination thereof being preferred in the gulumannuronate tablet, capsule or ampoule formulation preparation.

In particular, oligomannuronate or oligoguluronate comprise 2 to 16 β-D-mannuronic acid or α-L-guluronic acid monomers, preferably 3 to 6 β-D-mannuronic acid or α-L-guluronic acid monomers, e.g., 3 or 4 or 5 or 6 β-D-mannuronic acid or α-L-guluronic acid monomers.

In preferred embodiments, the tablet, capsule or ampoule formulation obtained by the method according to the invention may comprise at least one further supplement and/or at least one further pharmaceutical agent, preferably selected from antioxidant agents, anticancer agents, vitamins, multivitamins, (pharmaceutical) minerals, (pharmaceutical) multiminerals, amino acids, fatty acids, herbs, carbohydrates, analgesic agents, anti-inflammatory agents and agents with anti-sensitivity properties or combinations thereof, without being limited thereto, as described above.

Therefore, it is preferred that the method according to the invention is, in various embodiments, a method for preparing an antioxidant tablet, capsule or ampoule formulation, an anticancer tablet, capsule or ampoule formulation, an antiaging tablet, capsule or ampoule formulation, an analgesic tablet, capsule or ampoule formulation, and anti-inflammatory tablet, capsule or ampoule formulation and/or a tablet, capsule or ampoule formulation with anti-sensitivity properties or combinations thereof, without being limited thereto.

It is further possible that the tablet, capsule or ampoule formulation obtained by the method according to the invention comprises at least one further ingredient such as one or more auxiliaries as described herein.

All embodiments and examples described herein for the tablet, capsule or ampoule formulation for oral and/or injectable use according to the invention also apply to the tablet, capsule or ampoule formulation for oral and/or injectable use in a method for treating neurodegeneration, inflammatory reactions, pain, cancer, aging, diabetes, and/or cadiopathy, and to methods for preparing such formulations, and *vice versa.*

Other embodiments are within the following non-limiting examples.

### Examples

### 1. Method of preparation of mannuronic acid, guluronic acid and gulumannuronate powders from sodium alginate.

Mannuronic acid, guluronic acid and gulumannuronate were synthesized according to the GMP criteria of the WHO for substance production.

To synthesize "β-D-mannuronic acid", "α-L-guluronic acid" and "gulumannuronate (alginate hydrolysate)", the alginic acid sodium salt (sodium alginate) was used. In a first step, sodium alginate (100 g) was dissolved gently in 1500 ml H₂SO₄ 20% at 0 °C. The solution was thoroughly stirred at room temperature and was then heated to 85 °C until its color changed from a cream color to light brown. The hydrolysate was cooled to room temperature and the precipitate separated by centrifugation (3700 g). This precipitate can be used as gulumannuronic acid/gulumannuronate (alginate hydrolysate). The precipitate was re-dissolved by neutralization using 1 M Na₂CO₃ solution. This solution was then adjusted to pH 2.85 using 0.5 M HCI and the precipitate again separated by centrifugation (3700 g). The precipitate was collected and washed once with distilled water. This precipitate (α-L-guluronic acid) was spread and dried in petri dishes to yield α-L-guluronic acid in powder form. Said powder can be used in guluronate tablet, capsule or ampoule formulations and/or in the mixture of β-D-mannuronic acid and α-L-guluronic acid for gulumannuronate tablet, capsule or ampoule preparation. The remaining supernatant of the L-guluronic acid precipitate was collected and adjusted to pH 1.0 using 0.5 M HCI. Following centrifugation (3700 g), the precipitate was collected and washed once with distilled water. The obtained precipitate (β-D-mannuronic acid) was spread and dried in petri dishes to yield β-D-mannuronic acid in powder form. This may be used in mannuronate tablet, capsule or ampoule preparation and/or in the mixture of β-D-mannuronic acid with α-L-guluronic acid for gulumannuronate tablet, capsule or ampoule preparation. The characteristics of α-L-guluronic acid and β-D-mannuronic acid and their purity were validated by Fourier Transform Infrared (FT-IR) spectroscopy and Carbon-13 Nuclear Magnetic Resonance (13C-NMR) spectroscopy.

The provided powders (mannuronic acid) and (guluronic acid) were stored at room temperature (22 - 26 °C) in a dry place under sterile conditions in order to prepare the tablet, capsule or ampoule formulation for oral and/or injectable use.

### 2. Preparation of tablet or capsule formulations comprising mannuronic acid powder, guluronic acid powder or gulumannuronic acid powder in combination with caffeic acid powder.

For the preparation of tablet or capsule formulations comprising caffeic acid in combination with mannuronic acid/mannuronate, guluronic acid/guluronate or gulumannuronic acid/gulumannuronate, 0.03 g (30 mg) of caffeic acid powder is added to 10 g of β-D-mannuronic acid powder or α-L-guluronic acid powder or their mixture (gulumannuronate powder). The powders are preferably compressed to tablets.

## Claims

1. A tablet, capsule or ampoule formulation for oral and/or injectable use, the formulation comprising
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) a mixture of β-D-mannuronic acid, an oligomer thereof, or a pharmaceutically acceptable salt thereof and α-L-guluronic acid, an oligomer thereof, or a pharmaceutically acceptable salt thereof, preferably gulumannuronate,
wherein the total amount of β-D-mannuronic acid or α-L-guluronic acid, or their mixture, oligomers thereof, or pharmaceutically acceptable salts thereof is 10 wt.-% to 100 wt.-% based on the total weight of the tablet or capsule or 20 wt.-% to 60 wt.-% based on the total weight of the ampoule formulation.

2. A tablet or capsule formulation for oral and/or injectable use, preferably for oral use, according to claim 1, the formulation comprising
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) a mixture of β-D-mannuronic acid, an oligomer thereof, or a pharmaceutically acceptable salt thereof and α-L-guluronic acid, an oligomer thereof, or a pharmaceutically acceptable salt thereof, preferably gulumannuronate,
wherein the total amount of β-D-mannuronic acid or α-L-guluronic acid, or their mixture, oligomers thereof, or pharmaceutically acceptable salts thereof is 10 wt.-% to 100 wt.-% based on the total weight of the tablet or capsule.

3. An ampoule formulation for oral and/or injectable use, preferably for injectable use, according to claim 1, the formulation comprising
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) a mixture of β-D-mannuronic acid, an oligomer thereof, or a pharmaceutically acceptable salt thereof and α-L-guluronic acid, an oligomer thereof, or a pharmaceutically acceptable salt thereof, preferably gulumannuronate,
wherein the total amount of β-D-mannuronic acid or α-L-guluronic acid, or their mixture, oligomers thereof, or pharmaceutically acceptable salts thereof is 20 wt.-% to 60 wt.-% based on the total weight of the ampoule formulation.

4. The tablet, capsule or ampoule formulation according to any one of claims 1 to 3, wherein
(i) the β-D-mannuronic acid, an oligomer thereof, or a pharmaceutically acceptable salt thereof is β-D-mannuronate, preferably selected from the group consisting of sodium β-D-mannuronate, potassium β-D-mannuronate, magnesium β-D-mannuronate, calcium β-D-mannuronate, or ammonium β-D-mannuronate and combinations thereof, more preferably sodium β-D-mannuronate; or
(ii) the α-L-guluronic acid, an oligomer thereof, or a pharmaceutically acceptable salt thereof is α-L-guluronate, preferably selected from the group consisting of sodium α-L-guluronate, potassium α-L-guluronate, magnesium α-L-guluronate, calcium α-L-guluronate, or ammonium α-L-guluronate and combinations thereof, more preferably sodium α-L-guluronate; or
(iii) the mixture of β-D-mannuronic acid and α-L-guluronic acid, oligomers thereof, or pharmaceutically acceptable salts thereof, preferably gulumannuronate, is selected from the group consisting of sodium, or potassium, or magnesium, or calcium, or ammonium β-D-mannuronate and α-L-guluronate and combinations thereof, most preferably sodium β-D-mannuronate and/or sodium α-L-guluronate; or
(iv) the mixture of β-D-mannuronic acid and α-L-guluronic acid, oligomers thereof, or pharmaceutically acceptable salts thereof, preferably gulumannuronate, is alginate hydrolysate (powder).

5. The tablet, capsule or ampoule formulation according to any one of claims 1 or 4, wherein β-D-mannuronic acid and/or α-L-guluronic acid, oligomers thereof, or pharmaceutically acceptable salts thereof are selected from oligomers of β-D-mannuronate and/or α-L-guluronate.

6. The tablet, capsule or ampoule formulation according to claim 5, wherein the oligomer of β-D-mannuronate and/or α-L-guluronate
(i) is the homooligomer of β-D-mannuronate and/or the homooligomer of α-L-guluronate;
(ii) is selected from the group consisting of sodium, or potassium, or magnesium, or calcium, or ammonium oligomannuronate and/or oligoguluronate and combinations thereof, preferably sodium oligomannuronate and/or sodium oligoguluronate; and/or
(iii) comprises or consists of 2 to 16 β-D-mannuronic acid monomers and/or 2 to 16 α-L-guluronic acid monomers.

7. The tablet, capsule or ampoule formulation according to any one of claims 1 to 6, wherein the total amount of
(i) β-D-mannuronic acid, an oligomer thereof, or a pharmaceutically acceptable salt thereof, or
(ii) α-L-guluronic acid, an oligomer thereof, or a pharmaceutically acceptable salt thereof, or
(iii) the mixture of β-D-mannuronic acid and α-L-guluronic acid, oligomers thereof, or pharmaceutically acceptable salts thereof,
in the tablet or capsule is 30 wt.-% to 100 wt.-%, preferably 50 wt.-% to 100 wt.-%, based on the total weight of the tablet or capsule, or
in the ampoule formulation is 30 wt.-% to 60 wt.-%, preferably 40 wt.-% to 60 wt.-%, more preferably 50 wt.-% to 60 wt.-%, based on the total weight of the ampoule formulation.

8. The tablet or capsule formulation according to claim 7, wherein the mixture of β-D-mannuronic acid and α-L-guluronic acid, oligomers thereof, or pharmaceutically acceptable salts thereof, preferably the gulumannuronate formulation, comprises or consists of 0.01 wt.-% to 99.99 wt.-% of β-D-mannuronic acid, an oligomer thereof, or a pharmaceutically acceptable salt thereof, and 0.01 wt.-% to 99.99 wt.-% of α-L-guluronic acid, an oligomer thereof, or a pharmaceutically acceptable salt thereof, with the proviso that the total amount of β-D-mannuronic acid, an oligomer thereof, or a pharmaceutically acceptable salt thereof and α-L-guluronic acid, an oligomer thereof, or a pharmaceutically acceptable salt thereof in the tablet or capsule formulation is at least 10 wt.-% and at most 100 wt.-%, based on the total weight of the tablet or capsule, preferably
the total amount of β-D-mannuronic acid, an oligomer thereof, or a pharmaceutically acceptable salt thereof is 0.1 wt.-% to 99.9 wt.-%, more preferably 1 wt.-% to 99 wt.-%, more preferably 10 wt.-% to 90 wt.-%, based on the total weight of the tablet or capsule formulation, and
the total amount of α-L-guluronic acid, an oligomer thereof, or a pharmaceutically acceptable salt thereof is 0.1 wt.-% to 99.9 wt.-%, preferably 1 wt.-% to 99 wt.-%, more preferably 10 wt.-% to 90 wt.-%, based on the total weight of the tablet or capsule formulation.

9. The ampoule formulation according to claim 7, wherein the mixture of β-D-mannuronic acid and α-L-guluronic acid, oligomers thereof, or pharmaceutically acceptable salts thereof, preferably the gulumannuronate formulation, comprises or consists of 0.01 wt.-% to 59.99 wt.-% of β-D-mannuronic acid, an oligomer thereof, or a pharmaceutically acceptable salt thereof, and 0.01 wt.-% to 59.99 wt.-% of α-L-guluronic acid, an oligomer thereof, or a pharmaceutically acceptable salt thereof, with the proviso that the total amount of β-D-mannuronic acid, an oligomer thereof, or a pharmaceutically acceptable salt thereof and α-L-guluronic acid, an oligomer thereof, or a pharmaceutically acceptable salt thereof in the ampoule formulation is at least 20 wt.-% and at most 60 wt.-%, based on the total weight of the ampoule formulation, preferably
the total amount of β-D-mannuronic acid, an oligomer thereof, or a pharmaceutically acceptable salt thereof is 0.1 wt.-% to 59.9 wt.-%, more preferably 1 wt.-% to 59 wt.-%, more preferably 10 wt.-% to 50 wt.-%, more preferably 20 wt.-% to 40 wt.-%, based on the total weight of the ampoule formulation, and
the total amount of α-L-guluronic acid, an oligomer thereof, or a pharmaceutically acceptable salt thereof is 0.1 wt.-% to 59.9 wt.-%, preferably 1 wt.-% to 59 wt.-%, more preferably 10 wt.-% to 50 wt.-%, more preferably 20 wt.-% to 40 wt.-%, based on the total weight of the ampoule formulation.

10. The tablet, capsule or ampoule formulation according to any one of claims 1 to 9, wherein the tablet, capsule or ampoule formulation comprises at least one further supplement and/or at least one further pharmaceutical agent, preferably the tablet or capsule formulation comprises at least one further supplement and/or at least one further pharmaceutical agent, selected from antioxidant agents, anticancer agents, vitamins, multivitamins, (pharmaceutical) minerals, (pharmaceutical) multiminerals, amino acids, fatty acids, herbs, carbohydrates, analgesic agents, anti-inflammatory agents and agents with anti-sensitivity properties or combinations thereof.

11. The tablet, capsule or ampoule formulation according to any one of claims 1 to 10, wherein the tablet, capsule or ampoule formulation further comprises at least one further ingredient, preferably selected from binders, fillers, builders, complexing agents, preservatives, coating agents, alkaline agents, acidifiers, sweeteners, colorants, acids and bases or combinations thereof.

12. The tablet or capsule formulation according to any one of claim 1 to 11, wherein the tablet or capsule formulation further comprises caffeic acid or a salt thereof, for example in powder form, preferably in an amount of 0.001 wt.-% to 1 wt.-%, more preferably 0.01 wt.-% to 0.5 wt.-%, more preferably 0.05 wt.-% to 0.3 wt.-%, based on the total weight of the tablet or capsule formulation.

13. The tablet, capsule or ampoule formulation according to any one of claims 1 to 12, wherein the formulation is a supplement or medication, preferably an antioxidant tablet, capsule or ampoule formulation.

14. The tablet, capsule or ampoule formulation for oral and/or injectable use according to any one of claims 1 to 13 in a method for treating (or preventing)
(i) neurodegeneration, preferably in MS and Alzheimer's diseases; and/or
(ii) inflammatory reactions, preferably in rheumatic diseases; and/or
(iii) pain, preferably in joints and/or muscles; and/or
(iv) cancer, preferably in breast and prostate cancers; and/or
(v) aging; and/or
(vi) diabetes; and/or
(vii) cadiopathy; preferably in heart arrhythmia.

15. A method for preparing a tablet, capsule or ampoule formulation according to any one of claims 1 to 14, preferably the method is for preparing an antioxidant tablet, capsule or ampoule formulation, the method comprising the steps of
providing a composition:
(i) comprising β-D-mannuronic acid, an oligomer thereof, or a pharmaceutically acceptable salt thereof, preferably β-D-mannuronate, in a total amount of 10 wt.-% to 100 wt.-% based on the total weight of the tablet or capsule, or 20 wt.-% to 60 wt.-% based on the total weight of the ampoule formulation; or
(ii) comprising α-L-guluronic acid, an oligomer thereof, or a pharmaceutically acceptable salt thereof, preferably α-L-guluronate, in a total amount of 10 wt.-% to 100 wt.-% based on the total weight of the tablet or capsule, or 20 wt.-% to 60 wt.-% based on the total weight of the ampoule formulation; or
(iii) comprising a mixture of β-D-mannuronic acid and α-L-guluronic acid, oligomers thereof, or pharmaceutically acceptable salts thereof, preferably gulumannuronic acid, gulumannuronate or oligomers thereof, in a total amount of 10 wt.-% to 100 wt.-% based on the total weight of the tablet or capsule, or 20 wt.-% to 60 wt.-% based on the total weight of the ampoule formulation.
